# EUROPÄISCHE PATENTSCHRIFT

(11) **EP 2 432 373 B1**
(45) Veröffentlichungstag und Bekanntmachung des Hinweises auf die Patenterteilung: **09.05.2018**
(21) Anmeldenummer: 10726009.3
(22) Anmeldetag: 17.05.2010
(51) Int. Cl.: A61B 3/00, A61B 3/10, A61B 3/107

(54) **VERFAHREN UND ANALYSESYSTEM ZUR MESSUNG EINER AUGENGEOMETRIE**
METHOD AND ANALYSIS SYSTEM FOR MEASURING A GEOMETRY OF THE EYE
PROCÉDÉ ET SYSTÈME D'ANALYSE POUR MESURER UNE GÉOMÉTRIE OCULAIRE

(30) Priorität: 18.05.2009 DE 102009021770
(43) Veröffentlichungstag der Anmeldung: 28.03.2012
(73) Patentinhaber: Oculus Optikgeräte GmbH, 35582 Wetzlar (DE)
(72) Erfinder: KÖST, Gert, 30159 Hannover (DE); STEINMÜLLER, Andreas, 35435 Wettenberg (DE)
(74) Vertreter: advotec.
(86) Internationale Anmeldenummer: PCT/EP2010/056737
(87) Internationale Veröffentlichungsnummer: WO 2010/133549

(56) Entgegenhaltungen:
- EP-A1- 2 020 205
- DE-A1-102005 062 238
- DE-A1-102007 017 599
- US-A- 5 387 951
- US-A1- 2002 171 804

## Beschreibung

Die Erfindung betrifft ein Verfahren und ein ophthalmologisches Analysesystem zur Messung einer Augengeometrie an einem zu untersuchenden Auge mit einem ersten interferometrischen Analysesystem und einen zweiten nicht interferometrischen Analysesystem, wobei mit dem ersten Analysesystem Relativabstände beschreibende Messdaten von auf einer Messachse befindlichen optischen Grenzflächen des Auges gewonnen werden, wobei mit dem zweiten Analysesystem zumindest ein Bilddatensatz von auf der Messachse befindlichen optischen Grenzflächen gewonnen wird, wobei eine Verarbeitungseinrichtung des ophthalmologischen Analysesystems die Messdaten und den Bilddatensatz verarbeitet.

Derartige Verfahren zur Messung einer Augengeometrie bzw. ophthalmologische Analysesysteme, welche zwei unterschiedliche Analysesysteme kombinieren, sind hinreichend bekannt. So ist beispielsweise aus dem Stand der Technik ein ophthalmologisches Analysesystem bekannt, welches aus einem Interferometer und einem bildgebenden Analysesystem gebildet ist. Das bildgebende Analysesystem dient im Wesentlichen der Bestimmung einer Position einer optischen Grenzfläche bzw. Cornea relativ zum Interferometer. Das Interferometer dient zur Bestimmung eines Abstands einer Retina relativ zum Interferometer, so dass aus beiden Messwerten eine Achslänge eines Auges ermittelt werden kann.

Ein Verfahren und eine Vorrichtung gemäß der Oberbegriffe der unabhängigen Ansprüche 1 bzw. 14 sind aus dem Dokument EP 2 020 205 A1 bekannt.

Grundsätzlich weisen nicht interferometrische, bildgebende Analysesysteme den Nachteil auf, dass diese gegenüber einem Interferometer vergleichsweise ungenau sind. Jedoch lassen sich mit bildgebenden Analysesystemen die optischen Grenzflächen eines Auges, wie zum Beispiel die Außenfläche und die Innenfläche der Cornea sowie eine Forder- und Rückseite der Linse ermitteln und beispielsweise in einer Schnittansicht darstellen. Die Relativabstände der optischen Grenzflächen zueinander können aus einem derart gewonnenen Bilddatensatz ermittelt werden.

Mit einem Interferometer ist eine Messwerterfassung vergleichsweise zeitaufwendiger, da dass Interferometer eine Messstrecke mit einem darauf befindlichen Messpunkt, wie beispielsweise eine optische Grenzfläche, abtasten muss. Dies kann beispielsweise durch Verschieben eine Spiegels bzw. Verändern einer Länge einer optischen Referenzstrecke des Interferometers erfolgen. Wird der Vorgang des Abtastens für eine lange, mehrere optische Grenzflächen umfassende Messstrecke durchgeführt, bedarf es eines vergleichsweise relativ hohen Zeitaufwandes. Auch können bei einer derartigen Messung nur die Abstände der optischen Grenzflächen entlang einer Messachse ermittelt werden und nicht, wie bei verschiedenen bildgebenden Analyseverfahren ein Teilausschnitt einer optischen Grenzfläche, wie beispielsweise ein Krümmungsradius einer Cornea.

Der vorliegenden Erfindung liegt daher die Aufgabe zugrunde, ein Verfahren zur Messung einer Augengeometrie bzw. ein ophthalmologisches Analysesystem vorzuschlagen, welches die Augengeometrie beschreibende Bildinformationen mit verbesserter Genauigkeit gewinnen kann.

Diese Aufgabe wird durch ein Verfahren mit den Merkmalen des Anspruchs 1 sowie durch ein ophthalmologisches Analysesystem mit den Merkmalen des Anspruchs 14 gelöst.

Das erfindungsgemäße Verfahren zur Messung einer Augengeometrie an einem zu untersuchenden Auge wird mit einem ophthalmologischen Analysesystem, umfassend ein erstes interferometrisches Analysesystem und ein zweites nicht interferometrisches Analysesystem, durchgeführt, wobei mit dem ersten Analysesystem Relativabstände beschreibende Messdaten von auf einer Messachse befindlichen optischen Grenzflächen des Auges gewonnen werden, wobei mit dem zweiten Analysesystem zumindest ein Bilddatensatz von auf der Messachse befindlichen optischen Grenzflächen gewonnen wird, wobei eine Verarbeitungseinrichtung des ophthalmologischen Analysesystems die Messdaten und den Bilddatensatz verarbeitet, und wobei mittels der Verarbeitungseinrichtung der Bilddatensatz mit den Messdaten korrigiert wird.

Durch die Korrektur des Bilddatensatzes auf Basis der Messdaten des Interferometers ist für den Bilddatensatz, und damit für eine geometrische Bilddarstellung und Auswertung, eine verbesserte Genauigkeit erzielbar, die im Wesentlichen der Genauigkeit des Interferometers entspricht. Die Verarbeitungseinrichtung umfasst geeignete Mittel zur Verarbeitung der Messdaten und des Bilddatensatzes sowie zur grafischen Ergebnisdarstellung. Die Verarbeitungseinrichtung erkennt die optischen Grenzflächen jeweils aus den Messdaten und dem Bilddatensatz und korrigiert den Bilddatensatz zumindest teilweise für den mit den Messdaten überlappenden Bereich des Bilddatensatzes. Das Messverfahren kann abschnittsweise oder für eine gesamte Achslänge eines Auges durchgeführt werden, wobei grundsätzlich alle aus dem Stand der Technik bekannten und geeigneten interferometrischen und nicht interferometrischen Analysesysteme für das Messverfahren eingesetzt werden können.

Das zweite Analysesystem kann aus einer Projektionseinrichtung und einer Beobachtungseinrichtung gebildet sein, wobei mit der Projektionseinrichtung definierte Bereiche des Auges beleuchtet werden, und wobei mit der Beobachtungseinrichtung ein Bilddatensatz des beleuchteten Bereichs gewonnen werden kann. Ein derartiger Aufbau eines zweiten Analysesystems ermöglicht eine einfache Gewinnung eines Bilddatensatzes von optischen Grenzflächen des Auges.

So kann als zweites Analysesystem ein Scheimpflugsystem mit der Projektionseinrichtung und der Beobachtungseinrichtung, welche nach der Scheimpflugregel relativ zueinander angeordnet sind, verwendet werden. Die Projektionseinrichtung kann eine Spaltbeleuchtung des Auges entlang der Sehachse umfassen, wobei dann eine nach dem Scheimpflugprinzip angeordnete Kamera den so beleuchteten Querschnittsbereich des Auges erfassen kann. Ein sich so ergebendes Längsschnittbild des Auges kann dann vorzugsweise die optischen Grenzflächen der Cornea und der Linse wiedergeben. Aus dem erhaltenden Bilddatensatz kann die Verarbeitungseinrichtung die Relativabstände der optischen Grenzflächen einfach berechnen.

Eine besonders genaue Korrektur des Bilddatensatzes wird möglich, wenn die Messachse entlang einer Projektionsebene des zweiten Analysesystems verlaufend angeordnet wird. Durch den Verlauf der Messachse durch die Projektionsebene wird ein direkter Vergleich der Relativabstände der mit dem ersten und zweiten Analysesystem ermittelten optischen Grenzflächen möglich. Vorzugsweise kann die Messachse mit der Sehachse des Auges übereinstimmen. Alternativ ist es aber auch möglich, je nach Lage der Projektionsebene relativ zur Sehachse, die Messachse in einem Abstand zur Sehachse in einem außermittigen bzw. peripheren Bereich einer Augenvorkammer anzuordnen. Dies kann besonders vorteilhaft sein, wenn besonders genaue Messwerte von diesem Bereich ermittelt werden sollen.

In einer einfachen Verfahrensvariante kann mittels der Verarbeitungseinrichtung die Korrektur des Bilddatensatzes nach einem Vergleich eines Relativabstandes von zumindest zwei optischen Grenzflächen des Bilddatensatzes mit einem Relativabstand derselben optischen Grenzflächen der Messdaten erfolgen. Da der sich aus dem Bilddatensatz ergebende Relativabstand vergleichsweise ungenau ist, kann der in den Messdaten enthaltenen Relativabstand als ein Referenzmaß dienen, nach dem der Bilddatensatz korrigiert bzw. verändert wird. Ein besonders genauer Bilddatensatz kann erhalten werden, wenn der Messdatensatz mehr als zwei optische Grenzflächen, beispielsweise alle im Bilddatensatz enthaltenen optischen Grenzflächen, oder zwei besonders weit voneinander entfernte optische Grenzflächen bzw. deren Position enthält, und die Verarbeitungseinrichtung diese zur Korrektur verwendet.

Der Bilddatensatz kann besonders einfach korrigiert werden, wenn eine Dimension des Bilddatensatzes durch die Messachse definiert wird, und der Bilddatensatz in dieser Dimension korrigiert wird. So kann der Bilddatensatz einfach in eine Richtung der Messachse, je nach notwendiger Korrektur, gestaucht oder gestreckt werden, bis sich eine Position der im Bilddatensatz enthaltenen optischen Grenzflächen mit einer Position der selben in den Messdaten enthaltenen Grenzflächen überdeckt. Für den Fall, dass mehr als zwei optische Grenzflächen für eine Korrektur herangezogen werden, ist es möglich die Stauchung oder Streckung des Bilddatensatzes auf Basis einer nicht linearen Funktion zu korrigieren.

Auch kann eine Mehrzahl von Bilddatensätzen in einer sequentiellen Abfolge gewonnen werden. Beispielsweise kann das zweite Analysesystem eine Reihe von gleichmäßig beabstandeten, parallelen Schnittbildern entlang einer quer zur Sehachse verlaufenden Linie gewinnen. Die Verarbeitungseinrichtung kann dann die einzelnen Bilddatensätze dieser Schnittbilder zu einem Bilddatensatz zusammenfügen, der eine dreidimensionale Darstellung eines Auges ermöglicht. In diesem Zusammenhang kann es vorteilhaft sein, wenn das erste Analysesystem jeweils mit der Aufnahme eines Schnittbilds bzw. Bilddatensatzes eine Messung zumindest einer optischen Grenzfläche durchführt. Dies erleichtert ein Zusammenführen der jeweiligen Bilddatensätze in Bezug auf einen so erhaltenen Referenzpunkt. Auch können mögliche, während der sequentiellen Erfassung der Bilddatensätze erfolgte Augenbewegungen wesentlich genauer korrigiert werden.

Bei einer weiteren Alternative einer sequentiellen Bilddatensatzgewinnung kann eine Projektionsebene des zweiten Analysesystems um eine Sehachse des Auges verschwenkt werden. Die Projektionsebene kann demnach um die Sehachse des Auges rotiert werden. So ist vorteilhaft eine besonders hohe Datendichte innerhalb eines Bereichs um die Sehachse gewinnbar. Mit Bezug auf die zuvor beschriebenen Alternativen zur Gewinnung von Messdaten kann in diesem Fall die Messachse mit der Sehachse übereinstimmen, so dass mit dem ersten Analysesystem nur eine einzelne Messung notwendig ist. Wenn die Messachse nicht mit der Sehachse übereinstimmt, kann die Messachse auf einem Kreisdurchmesser koaxial zur Sehachse, den jeweiligen Projektionsebenen folgend, positioniert werden. Hieraus kann sich ein noch weiter verbessertes, dreidimensionales Bild mit geometrischen Abmessungen des Auges ergeben.

Weiter ist es möglich mittels des ersten Analysesystems eine Kalibrierung des zweiten Analysesystems durchzuführen. In der einfachsten Variante kann eine einmalige Korrektur eines ersten Bilddatensatzes mittels der Messdaten erfolgen, wobei alle möglichen, weiteren Bilddatensätze ebenfalls auf Basis der einmalig ermittelten Korrekturwerte korrigiert bzw. angepasst werden können.

Besonders vorteilhaft ist es, wenn das zweite Analysesystem einen ersten Bilddatensatz gewinnt, wobei auf Basis des Bilddatensatzes eine Relativposition zumindest einer optischen Grenzfläche als ein Referenzpunkt für das erste Analysesystem bestimmt wird. Das erste Analysesystem kann dann zielgerichtet die dann aus dem ersten Bilddatensatz bekannten optischen Grenzflächen abtasten, wodurch lange Abtastwege vermieden werden. Durch den so eingeschränkten Untersuchungsbereich des ersten Analysesystems kann die Erfassung der notwendigen Messdaten wesentlich beschleunigt werden.

Eine weitere Zeitoptimierung des Verfahrens wird ermöglicht, wenn die Messdaten und der Bilddatensatz gleichzeitig erfasst werden. Eine Korrektur des Bilddatensatzes kann dann unmittelbar nach Erhalt der Daten erfolgen.

Das erste Analysesystem und das zweite Analysesystem kann jeweils elektromagnetische Strahlung mit voneinander verschiedenen Wellenlängenbereichen aussenden. Die elektromagnetische Strahlung kann vorzugsweise im sichtbaren oder Infrarot-Wellenlängenbereich liegen. Eine die Datengewinnung nachteilig beeinflussende Überlagerung der Wellenlängenbereiche des ersten und zweiten Analysesystems kann so vorteilhaft vermieden werden.

Für ein erstes Analysesystem kann ein Michelson-Interferometer verwendet werden. Ein derartiges Interferometer hat sich für eine Verwendung mit dem Verfahren als besonders geeignet erwiesen.

Das erfindungsgemäße ophthalmologische Analysesystem zur Messung einer Augengeometrie an einem zu untersuchenden Auge umfasst ein erstes interferometrisches Analysesystem und ein zweites nicht interferometrisches Analysesystem, wobei mit dem ersten Analysesystem Relativabstände beschreibende Messdaten von auf einer Messachse befindlichen optischen Grenzflächen des Auges gewinnbar sind, wobei mit dem zweiten Analysesystem zumindest ein Bilddatensatz von auf der Messachse befindlichen optischen Grenzflächen gewinnbar ist, wobei das ophthalmologische Analysesystem eine Verarbeitungseinrichtung zur Verarbeitung der Messdaten und des Bilddatensatzes umfasst, und wobei die Verarbeitungseinrichtung so ausgebildet ist, dass der Bilddatensatz mit den Messdaten korrigiert werden kann.

Besonders vorteilhaft ist es, wenn das erste und das zweite Analysesystem in einem gemeinsamen Gehäuse angeordnet sind. Durch deren gemeinsame Nutzung lässt sich eine Vielzahl von Bauteilen einsparen. Beispielsweise müssen das Gehäuse selbst, notwendige Anbauteile, ein Netzteil sowie einige optische und elektronische Baugruppen dann nur einmal vorhanden sein.

Weitere vorteilhafte Ausführungsformen des ophthalmologischen Analysesystems ergeben sich aus den Merkmalsbeschreibungen der auf den Verfahrensanspruch 1 rückbezogenen Unteransprüche.

Im Folgenden wird die Erfindung unter Bezugnahme auf die beigefügte Zeichnung näher erläutert.

Es zeigen:
- **Fig. 1**: eine schematische Darstellung eines Aufbaus eines ophthalmologischen Analysesystems;
- **Fig. 2**: eine Vorderansicht eines zu analysierenden Auges;
- **Fig. 3**: eine Schnittansicht des zu analysierenden Auges.

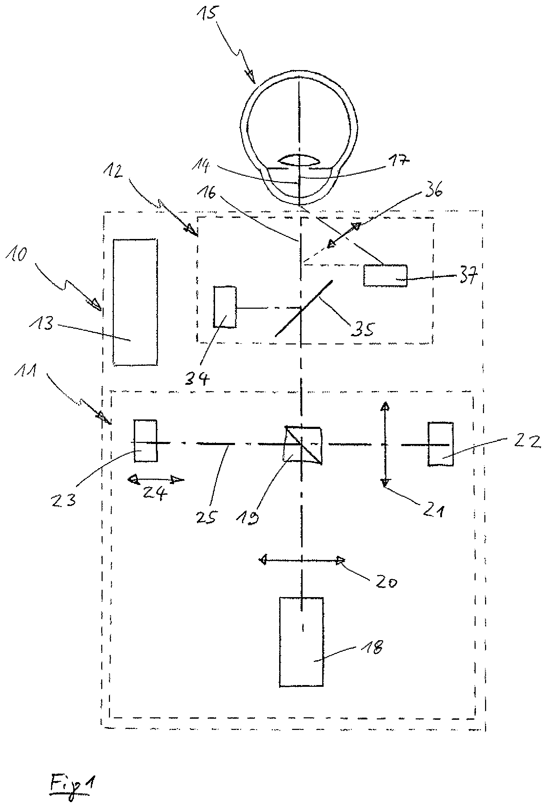

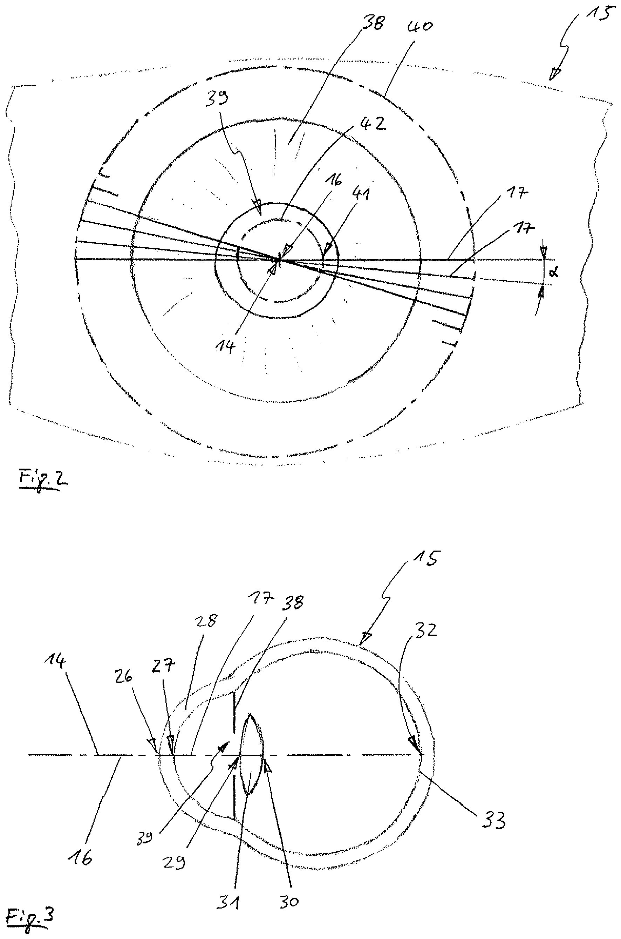

**Fig. 1** zeigt eine schematische Darstellung eines Aufbaus eines ophthalmologischen Analysesystems 10 umfassend ein als Interferometer 11 ausgebildetes erstes interferometrisches Analysesystem und ein als Scheimpflugaufnahmesystem 12 ausgebildetes zweites, nicht interferometrisches Analysesystem sowie eine Verarbeitungseinrichtung 13. Das ophthalmologische Analysesystem 10 ist relativ zu einer Sehachse 14 eines zu untersuchenden Auges 15 so angeordnet, dass die Sehachse 14 mit einer Messachse 16 des Interferometers 11 und eine Projektionsebene 17 des Scheimpflugaufnahmesystems 12 übereinstimmt bzw. diese durchläuft. Das Interferometer 11 ist im Wesentlichen aus einer Laserlichtquelle 18, einem optischen Teilerwürfel 19, Linsenanordnungen 20 und 21, einer Detektoreinrichtung 22 und einer Spiegeleinrichtung 23 gebildet. Insbesondere die Spiegeleinrichtung 23 ist längsverschieblich entlang des Doppelpfeils 24 angeordnet, so dass eine Länge einer Referenzstrecke 25 veränderlich ist. Durch eine Verschiebung der Spiegeleinrichtung 23 können verschiedene, auf der Sehachse 14 liegende Bereiche des Auges 15 abgetastet werden. Auf eine weitergehende Erläuterung einer bekannten Funktion des Interferometers 11 wird hier verzichtet. Insbesondere können Messdaten gewonnen werden, die Relativpositionen optischer Grenzflächen auf der Achse 14, wie eine vordere Fläche 26 einer Cornea 28, eine hintere Fläche 27 der Cornea 28, eine vordere Fläche 29 einer Linse 31, eine hintere Fläche 30 der Linse 31 und eine Fläche 32 einer Retina 33, gemäß der Darstellung in Fig. 3, beschreiben.

Das Scheimpflugaufnahmesystem 12 umfasst eine Spaltbeleuchtungseinrichtung 34, einen teiltransparenten Spiegel 35, eine Linsenanordnung 36 und eine Kameraeinrichtung 37. Ein hier nicht näher dargestellter Lichtspalt wird mittels des Spiegels 35 in Übereinstimmung mit der Sehachse 14 bzw. der Messachse 16 in das Auge 15 projiziert, so dass in der Projektionsebene 17 innerhalb des Auges 15 dessen transparente Bestandteile durch Lichtstreuung sichtbar gemacht werden können. Relativ zur Projektionsebene 17 ist nach der Scheimpflugregel die Linsenanordnung 36 und die Kameraeinrichtung 37 so angeordnet, dass ein hier nicht dargestelltes Bild der Projektionsebene 17 von der Kameraeinrichtung 37 als scharfe Abbildung erfasst und in einen Bilddatensatz umgewandelt werden kann.

Die mit dem Interferometer 11 ermittelten Messdaten der Flächen 26, 27 und 29, 30 sowie 32 werden der Verarbeitungseinrichtung 13 zugeleitet, wobei diese die Relativabstände der Flächen 26, 27 und 29, 30 sowie 32, bezogen auf die Messachse 16 bzw. die Sehachse 14, berechnet. Der mittels des Scheimpflugaufnahmesystems 12 gewonnene Bilddatensatz wird ebenfalls der Verarbeitungseinrichtung 13 zugeleitet, wobei diese mittels Bildverarbeitung eine Position der Flächen 26, 27 und 29, 30 und gegebenenfalls 32 relativ zur Messachse 16 bzw. Sehachse 14 berechnet. Da die auf Basis der Messdaten berechneten Relativabstände vergleichsweise genauer sind als die auf Basis des Bilddatensatzes berechneten Relativabstände, wird der Bilddatensatz von der Verarbeitungseinrichtung 13 so korrigiert, dass die Relativabstände des Bilddatensatzes mit den Relativabständen der Messdaten übereinstimmen. Insbesondere ist dabei vorgesehen den Bilddatensatz in Richtung der Messachse 16 bzw. Sehachse 14 so zu verändern, dass eine Stauchung bzw. Dehnung der Abbildung erfolgt. Die vorbeschriebene Korrektur kann auch alleine auf Basis eines einzelnen Relativabstandes erfolgen.

**Fig. 2** zeigt eine abschnittsweise Vorderansicht des Auges 15 bei der eine Iris 38 mit einer Pupillenöffnung 39 erkennbar ist. Weiter ist die Projektionsebene 17 als eine horizontale Linie dargestellt, durch die die Sehachse 14 verläuft. Das Scheimpflugaufnahmesystem 12 ist so ausgebildet, dass die Projektionsebene 17 nach Gewinnung eines ersten Bilddatensatzes in einem Winkel α um die Sehachse 14 zur Gewinnung eines zweiten Bilddatensatzes und n weiteren Bilddatensätzen in einer sequentiellen Abfolge verschwenkt werden kann. Die Gewinnung der Bilddatensätze erfolgt innerhalb eines durch die Projektionsebene 17 beschriebenen Kreisdurchmessers 40. Die Messachse 16 fällt wie vorbeschrieben in die Sehachse 14, so dass alle gewonnenen Bilddatensätze mit einem einzelnen Satz Messdaten korrigiert werden können. Alternativ ist in der **Fig. 2** eine Messachse 41 dargestellt, die mit der Projektionsebene 17 in dem Winkel α auf einem Kreisdurchmesser 42 um die Sehachse 14 verschwenkt wird. Diese Ausführungsform bedingt ein hier nicht dargestelltes optisches Analysesystem mit einer entsprechenden Anordnung eines Interferometers bzw. einer Strahlumlenkung der Messachse 41. Die so gewonnenen Messdaten für jeweils einen Bilddatensatz ermöglichen eine korrigierte, räumlich Darstellung bzw. geometrische Erfassung einer Augentopographie in dem von dem Interferometer abgetasteten Bereich um die Sehachse 14.

## Patentansprüche

1. Verfahren zur Messung einer Augengeometrie an einem zu untersuchenden Auge (15) mit einem ophthalmologischen Analysesystem (10) umfassend ein erstes interferometrisches Analysesystem (11) und ein zweites nicht interferometrisches Analysesystem (12), wobei mit dem ersten Analysesystem Relativabstände beschreibende Messdaten von auf einer Messachse (16, 41) befindlichen optischen Grenzflächen (26, 27, 29, 30, 32) des Auges gewonnen werden, wobei mit dem zweiten Analysesystem zumindest ein Bilddatensatz von auf der Messachse befindlichen optischen Grenzflächen des Auges gewonnen wird, wobei eine Verarbeitungseinrichtung (13) des ophthalmologischen Analysesystems die Messdaten und den Bilddatensatz verarbeitet,
**dadurch gekennzeichnet,**
**dass** mittels der Verarbeitungseinrichtung der Bilddatensatz mit den Messdaten korrigiert wird, wobei die Verarbeitungseinrichtung dazu eingerichtet ist, mittels Bildverarbeitung eine Position der optischen Grenzflächen auf der Messachse jeweils aus Messdaten und Bilddatensatz relativ zur Messachse (16, 41) zu erkennen und Relativabstände der Grenzflächen des Bilddatensatzes und der Messdaten zu berechnen und den Bilddatensatz für einen mit den Messdaten überlappenden Bereich so zu korrigieren, dass die Relativabstände des Bilddatensatzes mit den Relativabständen der Messdaten übereinstimmen.

2. Verfahren nach Anspruch 1,
**dadurch gekennzeichnet,**
**dass** das zweite Analysesystem (12) aus einer Projektionseinrichtung (34, 35) und einer Beobachtungseinrichtung (36, 37) gebildet ist, wobei mit der Projektionseinrichtung definierte Bereiche des Auges (15) beleuchtet werden, und wobei mit der Beobachtungseinrichtung ein Bilddatensatz des beleuchteten Bereichs gewonnen wird.

3. Verfahren nach Anspruch 2,
**dadurch gekennzeichnet,**
**dass** als zweites Analysesystem (12) ein Scheimpflugsystem mit der Projektionseinrichtung (34, 35) und der Beobachtungseinrichtung (36, 37), welche nach der Scheimpflugregel relativ zueinander angeordnet sind, verwendet wird.

4. Verfahren nach einem der vorangehenden Ansprüche,
**dadurch gekennzeichnet,**
**dass** die Messachse (16, 41) entlang einer Projektionsebene (17) des zweiten Analysesystems (12) verlaufend angeordnet wird.

5. Verfahren nach einem der vorangehenden Ansprüche,
**dadurch gekennzeichnet,**
**dass** die Korrektur des Bilddatensatzes nach einem Vergleich eines Relativabstandes von zumindest zwei optischen Grenzflächen (26, 27, 29, 30, 32) des Bilddatensatzes mit einem Relativabstand der selben optische Grenzflächen der Messdaten erfolgt.

6. Verfahren nach einem der vorangehenden Ansprüche,
**dadurch gekennzeichnet,**
**dass** eine Dimension des Bilddatensatzes durch die Messachse (16, 41) definiert wird, und der Bilddatensatz in dieser Dimension korrigiert wird.

7. Verfahren nach einem der vorangehenden Ansprüche,
**dadurch gekennzeichnet,**
**dass** eine Mehrzahl Bilddatensätze in einer sequentiellen Abfolge gewonnen wird.

8. Verfahren nach Anspruch 7,
**dadurch gekennzeichnet,**
**dass** eine Projektionsebene (17) des zweiten Analysesystems (12) um eine Sehachse (15) des Auges (15) verschwenkt wird.

9. Verfahren nach einem der vorangehenden Ansprüche,
**dadurch gekennzeichnet,**
**dass** mittels des ersten Analysesystems (11) eine Kalibrierung des zweiten Analysesystems (12) durchgeführt wird.

10. Verfahren nach einem der vorangehenden Ansprüche,
**dadurch gekennzeichnet,**
**dass** das zweite Analysesystem (12) einen ersten Bilddatensatz gewinnt, wobei auf Basis des Bilddatensatzes eine Relativposition zumindest einer optischen Grenzfläche (26, 27, 29, 30, 32) als ein Referenzpunkt für das erste Analysesystem (11) bestimmt wird.

11. Verfahren nach Anspruch 9,
**dadurch gekennzeichnet,**
**dass** die Messdaten und der Bilddatensatz gleichzeitig erfasst werden.

12. Verfahren nach einem der vorangehenden Ansprüche,
**dadurch gekennzeichnet,**
**dass** erste Analysesystem (11) und das zweite Analysesystem (12) jeweils
elektromagnetische Strahlung mit voneinander verschiedenen Wellenlängenbereichen aussenden.

13. Verfahren nach einem der vorangehenden Ansprüche,
**dadurch gekennzeichnet,**
**dass** als erstes Analysesystem (11) ein Michelson Interferometer verwendet wird.

14. Ophthalmologisches Analysesystem (10) zur Messung einer Augengeometrie an einem zu untersuchenden Auge (15) mit einem ersten interferometrischen Analysesystem (11) und einem zweiten nicht interferometrischen Analysesystem (12), wobei das erste Analysesystem dazu eingerichtet ist, Relativabstände beschreibende Messdaten von auf einer Messachse (16, 41) befindlichen optischen Grenzflächen (26, 27, 29, 30, 32) des Auges zu gewinnen, wobei das zweite Analysesystem dazu eingerichtet ist, zumindest ein Bilddatensatz von auf der Messachse befindlichen optischen Grenzflächen des Auges zu gewinnen, wobei das ophthalmologische Analysesystem eine Verarbeitungseinrichtung (13) eingerichtet zur Verarbeitung der Messdaten und des Bilddatensatzes umfasst,
**dadurch gekennzeichnet,**
**dass** die Verarbeitungseinrichtung so ausgebildet ist, dass der Bilddatensatz mit den Messdaten korrigiert wird, wobei die Verarbeitungseinrichtung dazu eingerichtet ist, mittels Bildverarbeitung eine Position der optischen Grenzflächen auf der Messachse jeweils aus Messdaten und Bilddatensatz relativ zur Messachse (16, 41) zu erkennen und Relativabstände der Grenzflächen des Bilddatensatzes und der Messdaten zu berechnen und den Bilddatensatz für einen mit den Messdaten überlappenden Bereich so zu korrigieren, dass die Relativabstände des Bilddatensatzes mit den Relativabständen der Messdaten übereinstimmen.

15. Analysesystem nach Anspruch 14,
**dadurch gekennzeichnet,**
**dass** das erste Analysesystem (11) und das zweite Analysesystem (12) in einem gemeinsamen Gehäuse angeordnet ist.

## Claims

1. A method for measuring a geometry of an eye on an eye (15) to be examined using an ophthalmological analysis system (10), comprising a first interferometric analysis system (11) and a second non-interferometric analysis system (12), measured data describing relative distances being obtained from optical boundary surfaces (26, 27, 29, 30, 32) of the eye on a measurement axis (16, 41) using the first analysis system, at least one image data set being obtained from optical boundary surfaces of the eye on the measurement axis using the second analysis system, a processing device (13) of the ophthalmological analysis system processing the measured data and the image data set,
**characterized in that**
the image data set is corrected by means of the processing device using the measured data, said processing device being configured for identifying a position of the optical boundary surfaces on the measurement axis from measured data and the image data set, respectively, relative to the measurement axis (16, 41) by means of image processing and for computing relative distances of the boundary surfaces of the image data set and the measured data and for correcting the image data set for an area, which overlaps with the measured data, in such a manner that the relative distances of the image data set correspond to the relative distances of the measured data.

2. The method according to claim 1,
**characterized in that**
the second analysis system (12) is made of a projection device (34, 35) and an observation device (36, 37), areas of the eye (15) defined by the projection device being illuminated, and an image data set of the illuminated area being obtained by using the observation device.

3. The method according to claim 2,
**characterized in that**
a Scheimpflug system having the projection device (34, 35) and the observation device (36, 37), which are arranged in relation to one another according to the Scheimpflug rule, is used as the second analysis system (12).

4. The method according to any one of the preceding claims,
**characterized in that**
the measurement axis (16, 41) is arranged so as to extend along a projection plane (17) of the second analysis system (12).

5. The method according to any one of the preceding claims,
**characterized in that**
the image data set is corrected after comparing a relative distance of at least two optical boundary surfaces (26, 27, 29, 30, 32) of the image data set to a relative distance of the same optical boundary surfaces of the measured data.

6. The method according to any one of the preceding claims,
**characterized in that**
a dimension of the image data set is defined by the measurement axis (16, 41) and the image data set is corrected in this dimension.

7. The method according to any one of the preceding claims,
**characterized in that**
a plurality of image data sets is obtained in sequential order.

8. The method according to claim 7,
**characterized in that**
a projection plane (17) of the second analysis system (12) is pivoted about an optical axis (14) of the eye (15).

9. The method according to any one of the preceding claims,
**characterized in that**
the second analysis system (12) is calibrated by means of the first analysis system (11).

10. The method according to any one of the preceding claims,
**characterized in that**
the second analysis system (12) obtains a first image data set, a relative position of at least one optical boundary surface (26, 27, 29, 30, 32) being determined as a reference point for the first analysis system (11) based on the image data set.

11. The method according to claim 9,
**characterized in that**
the measured data and the image data set are detected at the same time.

12. The method according to any one of the preceding claims,
**characterized in that**
the first analysis system (11) and the second analysis system (12) each emit electromagnetic radiation of different wavelength ranges.

13. The method according to any one of the preceding claims,
**characterized in that**
a Michelson interferometer is used as the first analysis system (11).

14. An ophthalmological analysis system (10) for measuring a geometry of an eye on an eye (15) to be examined using a first interferometric analysis system (11) and a second non-interferometric analysis system (12), said first analysis system being configured for obtaining measured data describing relative distances from optical boundary surfaces (26, 27, 29, 30, 32) of the eye on a measurement axis (16, 41), said second analysis system being configured for obtaining at least one image data set of optical boundary surfaces of the eye on the measurement axis, said ophthalmological analysis system comprising a processing device (13) configured for processing the measured data and the image data set,
**characterized in that**
the processing device is designed such that the image data set is corrected using the measured data, said processing device being configured for identifying a position of the optical boundary surfaces on the measurement axis from measured data and image data sets, respectively, relative to the measurement axis (16, 41) by means of image processing and for computing relative distances of the boundary surfaces of the image data set and the measured data and for correcting the image data set for an area, which overlaps with the measured data, in such a manner that the relative distances of the image data set correspond to the relative distances of the measured data.

15. An analysis system according to claim 14,
**characterized in that**
the first analysis system (11) and the second analysis system (12) are arranged in a shared housing.

## Revendications

1. Procédé pour mesurer une géométrie d'oeil à un oeil (15) à être analysé en utilisant un système (10) d'analyse ophtalmologique, comprenant un premier système (11) d'analyse interférométrique et un deuxième système (12) d'analyse non interférométrique, des données de mesure décrivant des distances relatives étant obtenues par des surfaces (26, 27, 29, 30, 32) de délimitation optiques de l'oeil disposées sur un axe (16, 41) de mesure en utilisant le premier système d'analyse, au moins un ensemble de données d'image des surfaces de délimitation optiques de l'oeil disposées sur l'axe de mesure étant obtenu en utilisant le deuxième système d'analyse, un dispositif (13) de traitement du système d'analyse ophtalmologique traitant les données de mesure et l'ensemble de données d'image,
**caractérisé en ce que**
l'ensemble de données d'image est corrigé au moyen du dispositif de traitement en utilisant les données de mesure, ledit dispositif de traitement étant configuré de manière à identifier une position des surfaces de délimitation optiques sur l'axe de mesure à partir des données de mesure et de l'ensemble de données d'image, respectivement, relatif à l'axe (16, 41) de mesure en utilisant le traitement d'image et de manière à calculer des distance relatives des surfaces de délimitation de l'ensemble de données d'image et des données de mesure et de manière à corriger l'ensemble de données d'image pour une zone, qui superpose les données de mesure, de telle manière que les distances relatives de l'ensemble de données d'image correspondent aux distances relatives des données de mesure.

2. Procédé selon la revendication 1,
**caractérisé en ce que**
le deuxième système (12) d'analyse est fait d'un dispositif (34, 35) de projection et un dispositif (36, 37) d'observation, des zones de l'oeil (15) étant illuminées par le dispositif de projection, et un ensemble de données d'image de la zone illuminée étant obtenu par le dispositif d'observation.

3. Procédé selon la revendication 2,
**caractérisé en ce qu'**
un système Scheimpflug ayant le dispositif (34, 35) de projection et le dispositif (36, 37) d'observation, qui sont disposés en relation de l'un à l'autre selon le loi de Scheimpflug, est utilisé en tant que deuxième système (12) d'analyse.

4. Procédé selon l'une quelconque des revendications précédentes,
**caractérisé en ce que**
l'axe (16, 41) de mesure est disposé de manière à s'étendre le long du plan (17) de projection du deuxième système (12) d'analyse.

5. Procédé selon l'une quelconque des revendications précédentes,
**caractérisé en ce que**
l'ensemble de données d'image est corrigé après une comparaison d'une distance relative d'au moins deux surfaces (26, 27, 29, 30, 32) de délimitation optiques de l'ensemble de données d'image avec une distance relative de la même surface de délimitation optique des données de mesure.

6. Procédé selon l'une quelconque des revendications précédentes,
**caractérisé en ce qu'**
une dimension de l'ensemble de données d'image est définie par l'axe (16, 41) de mesure, et l'ensemble de données d'image est corrigé dans cette dimension.

7. Procédé selon l'une quelconque des revendications précédentes,
**caractérisé en ce qu'**
une pluralité d'ensemble de données d'image est obtenue dans une succession séquentielle.

8. Procédé selon la revendication 7,
**caractérisé en ce qu'**
un plan (17) de projection du deuxième système (12) d'analyse est pivoté autour d'un axe (15) optique de l'oeil (15).

9. Procédé selon l'une quelconque des revendications précédentes,
**caractérisé en ce que**
le deuxième système (12) d'analyse est calibré au moyen du premier système (11) d'analyse.

10. Procédé selon l'une quelconque des revendications précédentes,
**caractérisé en ce que**
le deuxième système (12) d'analyse obtient un premier ensemble de données d'image, une position relative d'au moins une surface (26, 27, 29, 30, 32) de délimitation optique est déterminée en tant que point de référence pour la premier système (11) d'analyse sur la base de l'ensemble de données d'image.

11. Procédé selon la revendication 9,
**caractérisé en ce que**
les données de mesure et l'ensemble de données d'image sont saisis au même temps.

12. Procédé selon l'une quelconque des revendications précédentes,
**caractérisé en ce que**
le premier système (11) d'analyse et le deuxième système (12) d'analyse émet chacun du rayonnement électromagnétique ayant des plages de longue d'onde ayant des longueurs différentes.

13. Procédé selon l'une quelconque des revendications précédentes,
**caractérisé en ce qu'**
un interféromètre Michelson est utilisé en tant que premier système (11) d'analyse.

14. Système (10) d'analyse ophtalmologique pour mesurer une géométrie d'oeil à un oeil (15) à être analysé en utilisant un premier système (11) d'analyse interférométrique et un deuxième système (12) d'analyse non interférométrique, ledit premier système d'analyse étant configuré de manière à obtenir des données de mesure décrivant des distances relatives à partir des surfaces (26, 27, 29, 30, 32) de délimitation optique de l'oeil disposées sur un axe (16, 41) de mesure, ledit deuxième système d'analyse étant configuré de manière à obtenir au moins un ensemble de données d'image à partir des surfaces de délimitation optiques de l'oeil disposées sur l'axe de mesure, ledit système d'analyse ophtalmologique comprenant un dispositif (13) de traitement configuré de manière à traiter les données de mesure et l'ensemble de données d'image,
**caractérisé en ce que**
le dispositif de traitement est réalisé de telle manière que l'ensemble de données d'image soit corrigé en utilisant les données de mesure, ledit dispositif de traitement étant configuré de manière à identifier une position des surfaces de délimitation optiques sur l'axe de mesure à partir des données de mesure et de l'ensemble de données d'image, respectivement, relatif à l'axe (16, 41) de mesure au moyen du traitement d'image et de manière à calculer des distances relatives des surfaces de délimitation de l'ensemble de données d'image et des données de mesure et de manière à corriger l'ensemble de données d'image pour une zone, qui superpose les données de mesure, de telle manière que les distances relatives de l'ensemble de données d'image correspondent aux distances relatives des données de mesure.

15. Système d'analyse selon la revendication 14,
**caractérisé en ce que**
le premier système (11) d'analyse et le deuxième système (12) d'analyse sont disposés dans une boîte commune.
